# EUROPEAN PATENT APPLICATION

(11) **EP 1 961 409 A2**
(43) Date of publication of application: **27.08.2008**
(21) Application number: 07024810.9
(22) Date of filing: 20.12.2007
(51) Int. Cl.: A61K 8/11, A61K 8/25, A61K 8/34, A61K 8/44, A61K 8/73, A61K 8/92, A61Q 19/10

(54) **Compressible solid particles, procedures for the obtention thereof, and procedures for the use of said solid particles in tablets for body cleansing**

(30) Priority: 20.12.2006 AR P060105647
(71) Applicant: Mumoli, José Alejandro, 1684 C.J. El Palomar, Buenos Aires (AR); Makuc, Roben Antonio, Ciudad de Buenos Aires (AR)
(72) Inventor: Mumoli, José Alejandro, 1684 C.J. El Palomar, Buenos Aires (AR); Makuc, Roben Antonio, Ciudad de Buenos Aires (AR)
(74) Representative: Wibbelmann, Jobst

(57) **Abstract**

Compressible solid tablets comprising from 20 to 38 wt% of an oily or viscous liquid, from 50 to 70 wt% of a cellulose polymer and from 2 to 15 wt% of an hydrophobic coating compound, wherein said particles size is 180 micrones or less. For example, the oily or viscous liquid may consist of plant oils or surfactants such as cocamide DEA, cocoamide propyl betaine and mixtures thereof. The cellulose polymer may be carboxy methyl cellulose, microcrystalline cellulose or mixtures thereof. The coating hydrophobic composition may be silicon dioxide, calcium orthophosphate, magnesium carbonate, aluminum oxide. Compressible solid particles are also disclosed which comprise from 50 to 70 wt% of a crystalline alcohol, from 25 to 45% of a cellulose polymer and from 5 to 18 wt% of a coating hydrophobic compound. For example, the crystalline alcohol may be a polyol such as xylitol, isomaltose, sorbitol, maltitol, starch hidrolisate; a terpene such as thymol, carvacrol or menthol. Particles are used in the manufacturing of tablets for personal hygiene in a quantity comprised from 1 to 16 wt% on the basis of the whole tablet.

## Description

This invention refers to compressible solid particles, procedures for the obtention thereof and procedures for the use of said solid particles in tablets for body cleansing. Compressible solid particles comprise from 20 to 38 wt% of an oily or viscous liquid, from 50 to 70 wt% of a cellulose polymer and from 2 to 15 wt% of an hydrophobic coating, wherein said particles size is 180 µ or less. For example, the oily or viscous liquid may consist of plant oils or surfactants such as cocamide DEA, cocoamide propyl betaine and mixtures thereof. The cellulose polymer may be carboxy methyl cellulose, microcrystalline cellulose or mixtures thereof. The coating hydrophobic composition may be silicon dioxide, calcium or magnesium stearates, calcium orthophosphate, calcic carbonate, colloidal silica, aluminum oxides.

Compressible solid particles are also disclosed which comprise from 50 to 70 wt% of a crystalline alcohol, from 25 to 45 wt% of a cellulose polymer and from 5 to 18 wt% of a coating hydrophobic compound. For example, the crystalline alcohol may be a polyol such as xylitol, isomaltose, sorbitol; a terpene such as thymol; carvacrol or menthol. Particles are used in the manufacturing of tablets for personal hygiene in a quantity comprised from 1 to 16 wt% on the basis of the whole tablet.

### BACKGROUND OF THE INVENTION

Within the procedure for the obtention of tablets through simple compression each of the components should satisfy certain requisites in order that the resulting product be compact and firm and also comply with the friability, hardness and disintegration speed characteristics desired for the objective hereof.

When it is the objective to obtain quickly disintegrating tablets, for example those disclosed in Patents Nos. US 65411441 B2 and US 6664225 B2 it is necessary that components thereof be capable of being compressed by simple or direct compression without modifying the resulting tablet characteristics. For example, the incorporation of essential oils or crystalline alcohols is particularly difficult. Adding of these components prevents the obtention of tablets capable of disintegrating quickly, for example in less than 30 seconds, and that said tablets further possess a friability of less than 1.2%.

Patent document WO 03/053410 discloses a method for the obtention of a particulate composition from a mixture wherein at least one of the compounds is liquid, the mixture comprising a lipophilic liquid, an hydrophilic solid disperser and a solid particulate agent. Lipophilic liquid may be an essential oil, the solid hydrophilic disperser may be cellulose, and the solid particulate agent may be particulate silicon dioxide. The obtention of particles forming part of a larger composition, for example solid particles which comprise from 1 to 16 wt% of the total weight of a tablet, is not an object of this patent document. Said document neither discloses the intention of obtaining personal hygiene tablets which are solid and quickly disintegrate upon contacting a liquid medium such as water.

Patent document ES 224471213 discloses a method for the production of high purity xylitol powder, said method is carried out spraying at temperatures higher than room temperature. When this xylitol is used in tablets, it is particularly disclosed that later the adding of CMC will not be necessary in order to reduce hardness of the obtained tablet.

In patent document ES 2242523 a compressible powder xylitol is disclosed but it lacks CMC or other hygroscopic compound capable of preserving its life. The patent document mentions that by adding dextrin, xylitol is afforded the hardness required for a tablet preparation. The intention is to obtain tablets bearing a high weight proportion of xylitol.

Patent document ES 2241334 discloses quick dissolving tablets which comprise xylitol as sweetener, an active principle such as ibuprofene and disintegrating agents such as croscarmellose. This palatable tablet would be quickly absorbed orally.

Direct compression procedures do not require previous granulation steps, the only requirement being that compounds should be directly compressible. In order to avoid many of the problems produced by direct compression, the pharmaceutical industry performs dry granulation or wet granulation procedures.

Cosmetic industry does not need methods for improving the obtention of tablets as there exist few compressible cosmetic preparations.

One of the greatest difficulties regarding the obtention of tablets is that of be able to compress all of the compounds in order to obtain a compressed product bearing all of the desired features. In some cases, the introduction of essential oils or crystals, for example crystalline alcohols, even in very small quantities, makes the simple procedure for the obtention of personal hygiene tablets a difficult task. For instance, crystalline alcohols such as xylitol produce clusters so large that it is not possible to compress all of the compounds in a uniform fashion, and the tablet obtained does not fulfil those properties relating to hardness and dissolution which are required from a personal hygiene tablet.

On the other hand, essential oils are frequently sprayed, and at temperatures higher than room temperature, making the compression procedure more complex and costly.

### BRIEF DESCRIPTION OF THE INVENTION

It is an object of the present invention the provision of compressible solid particles comprising from 20 to 38 wt% of an oily or viscous liquid, from 50 to 70 wt% of a cellulose polymer and from 2 to 15 wt% of a coating hydrophobic compound, wherein the size of said particles is of 30 microns or less. For example, the oily or viscous liquid may be a vegetable oil or a surfactante such as cocamide DEA, cocoamide propyl betaine and mixtures thereof. The cellulose polymer may be carboxy methyl cellulose, microcrystalline cellulose or mixtures thereof. The coating hydrophobic composition may be silicon dioxide, calcium or magnesium stearate, calcium orthophosphate, calcium or magnesium carbonate, coloidal silica or aluminum oxide.

It is another object of the present invention to provide a procedure for the obtention of said compressible solid particles which comprises the following stages:
i) contacting an oily liquid with a cellulose polymer;
ii) mixing of both components;
iii) addition of a coating hydrophobic composition;
iv) stirring of the coated mixture obtained in iii); and
v) sieving of the compressible solid particles, wherein said particles size is of 180 microns or less. Said procedure is performed at room temperature.

It is another object of the present invention to provide a procedure to obtain topic use tablets, which procedure comprises compression of a mixture comprising from 1 to 16 wt% of compressible solid particles and from 99 to 84 wt% of excipients, wherein said tablets friability is equal to 1.2% or less and their velocity of disintegration in water is equal to 30 seconds or less. Said compressible solid particles comprise from 20 to 38 wt% of an oily or viscous liquid, from 50 to 70 wt% of a cellulose polymer and from 2 to 15 wt% of a coating hydrophobic compound, wherein said particles size is equal to 180 microns or less.

It is still another object of the present invention the provision of compressible solid particles comprising from 50 to 70 wt% of a crystalline alcohol, from 25 to 45 wt% of a cellulose polymer and from 5 to 18 wt% of a coating hydrophobic compound. For example, the crystalline alcohol may be a polyol such as xylitol, isomaltose, sorbitol, maltitol, starch hydrolisates; a terpene such as thymol, carvacrol or menthol.

It is still another object of the present invention a procedure for the obtention of said compressible solid particles which comprises the following stages:
i) contacting said crystalline alcohol with a coating hydrophobic composition;
ii) mixing of both ingredients;
iii) addition of a cellulose polymer and further mixing; and
iv) sieving of the compressible solid particles, wherein said particles size is of 180 microns or less. Said compressible solid particles comprise from 50 to 70 wt% of a crystalline alcohol, from 25 to 45 wt% of a cellulose polymer and from 5 to 18 wt% of a coating hydrophobic compound, and the size of said particles is of 180 microns or less and said crystalline alcohols may consist of polyols, terpenes or menthol.

It is still another object of the present invention to provide a procedure for the obtention of topic use tablets which comprises the compression of a mixture comprising from 1 to 16 wt% of compressible solid particles and from 99 to 84 wt% of excipients, wherein said tablets exhibit a friability of 1.2% or less and a velocity of disintegration in water of 30 seconds or less and wherein said compressible solid tablets comprise from 50 to 70 wt% of a crystalline alcohol, from 25 to 45 wt% of a cellulose polymer and from 5 to 18 wt% of a coating hydrophobic compound, wherein said particles size is of 180 microns or less.

### DETAILED DESCRIPTION OF THE INVENTION

It is the object of the present invention to obtain solid particles smaller than 180 microns which are compressible from oily and/or viscous liquid compounds in dry formulations, as well as from solids -such as crystalline alcohols, in order to finally obtain a compressible solids compounds mixture for the obtention of tablets for personal hygiene.

Particles of the present invention are useful as raw materials or actives for topic use cosmetic and/or pharmaceutical formulations which are not edible and preferably consist of formulations for personal hygiene, such as: soaps, shampoos, dental pastes, shaving creams, etc., which are formed as tablets. In order to prepare tablets for pesonal hygiene it is preferable required from starting materials which are in the form of viscous or oily liquids, or agglomerating crystals such as for example crystalline alchohols be initially employed as a powder in order to introduce them into a mixture containing the rest of the majority of compounds. In fact, solid particles of the invention are added to a mixture of components in a quantity ranging from 1 to 16 w% with respect to the end mixture.

Preferably, the invention refers to the formulations and methodology to transform such raw materials, ingredients or actives to the form of powders which are compatible with the end formulations.

As in the cosmetic, phytocosmetic and phytotherapic fields tablets are not massively used, there is a lack of development of certain raw materials in the form of powder solids in more complex dry mixtures and then compress the obtained mixture into tablets as an end product. Such end tablets should have a friability of less than 1.2% g and a disintegration when in contact with a liquid medium of 30 seconds or less.

In a preferred embodiment, solid particles obtained by applying the procedure explained in Example 1 may be used in a quantity ranging from 1 to 16 wt% of total weight for the production of shampoo, foam baths or other objects in solid tablets.

In another preferred embodiment, solid particles obtained by applying the procedure explained in Example 2 may be used in a quantity ranging from 1 to 16 wt% of total weight for the production of dental paste, shaving cream or refreshing creams in solid tablets.

In another preferred embodiment, solid particles obtained by applying the procedure explained in Example 1 may be used in a quantity ranging from 1 to 16 wt% of total weight for the production of shampoo, foam baths or other objects in solid tablets for personal hygiene requiring perfume.

This invention will be further illustrated by means of the following examples, which are not to be considered a limitation to the scope hereof. On the contrary, it is to be clearly understood that many other embodiments, modifications and equivalents may be suggested to the expert in the art by reading this description, without departing from the spirit of the present invention and/or the scope of the accompanying claims.

### EXAMPLES

Example 1: Obtention of solid particles according to the invention from viscous liquids.

All of the components are to be weighted.
1) In an adequate container, mix 500 ml of COCAMIDE DEA and 250 ml of COCOAMIDE PROPYL BETAINE and stir with a low revolutions stirrer until an emulsion of the components is generated.
2) In a small lab mixer place 2.00 kg of microcrystalline cellulose PH 102 and 200 g of modified carboxy methyl cellulose, and mix during 5 minutes.
3) Slowly pour, with the mixer in operation, the mixture of liquids of step 1) kneading until homogeneous. Let it rest.
4) Place the mass in a grinder of adequate capacity with high speed cutting blades (1500/2800 rpm); grind all of the contents during about 5 minutes.
5) Add to the preparation 100 g of hydrophobic coloidal silicon dioxide and switch on again the cutting stirrer during 2 minutes, checking to be sure the container is tightly closed. After a rest of 5-10 minutes, transfer the contents to a plastic container.
6) Sieve with US 20 mesh and keep in closed containers.

EXAMPLE 2: Obtention of the solid particles according to the invention from crystalline alcohols

Weight all of the components.
1) In a grinder fitted with high speed (2800 rpm) cutting stirrer, lid and of an adequate volume, place 500 g of D-Xylitol crystals, grind until a powder of particle size distribution ranging from 50 to 90 microns is obtained.
2) Once the desired crystal size is obtained, in a small lab mixer, double cone, "V" or stirrer type, add 50 g of hydrophobic amorphous coloidal silicon dioxide to the grinded D-Xylitol, mix during 2/5 minutes and let it rest. Add 100 g of sodium croscarmellose and 250 g of microcrystalline cellulose PH 101. Mix during 10 minutes.
3) Sieve said mixture through US 20 mesh.
4) Store the end mixture in an adequate hermetic container.

EXAMPLE 3: Obtention of the solid particles according to the invention from essential oils
1) Weight all of the components.
2) In a boiler type mixer of adequate capacity introduce 5 kg of microcrystalline cellulose PH 101 and 250 g of sodium croscarmellose. Mix during 15 minutes.
3) By means of a spray type pistol of adjustable flow rate and fan, spray the previous mixture through the boiler mouth and with the later operating, with 3 liters of essential oils, during about 25 minutes until a totally impregnated mass is obtained by mixing continuosly.
4) With the boiler still operating, slowly add 200 g of coloidal silicon dioxide over the mixture. Cover and continue mixing during 15 minutes.
5) The powder obtained during the previous procedure (about 8.45 kg) is poured into an adequate container, all of the mixture is sieved through US 30 mesh and store the obtained powder product hermetically.

## Claims

1. Compressible solid particle **characterized in that** they comprise from 20 to 38 wt% of an oily or viscous liquid, from 50 to 70 wt% of a cellulose polymer and from 2 to 15 wt% of an hydrophobic coating compound, wherein said particles size is 180 microns or less.

2. The particle according to claim 1, **characterized in that** the oily or viscous liquid is selected from plant oils and surfactants.

3. The particle according to claim 2, **characterized in that** the surfactant is selected from the group consisting of cocamide DEA, cocoamide propyl betaine and mixtures thereof.

4. The particle according to claim 1, **characterized in that** the cellulose polymer is selected from the group comprising carboxy methyl cellulose, microcrystalline cellulose and mixtures thereof.

5. The particle according to claim 1, **characterized in that** the coating hydrophobic composition comprises compounds selected from the group comprising silicon dioxide, calcium orthophosphate, calcium carbonate, magnesium carbonate, aluminum oxide, coloidal silica.

6. A procedure for the obtention of the compressible solid particles according to claim 1, **characterized in that** it comprises the stages of:
vi) contacting an oily liquid with a cellulose polymer;
vii) mixing of both components;
viii) addition of a coating hydrophobic composition to said mixture;
ix) stirring of the coated mixture obtained in iii); and
x) sieving of the compressible solid particles, wherein said particles size is of 180 microns or less.

7. The procedure according to claim 6, **characterized in that** the oily liquid is selected from the group comprised by plant oils and surfactants.

8. The procedure acccording to claim 7, **characterized in that** the surfactant is selected from the group comprised by cocamide DEA, coco amide propyl betaine and mixtures thereof.

9. The procedure according to claim 6, **characterized in that** the cellulose polymer is selected from the group comprised of carboxy methyl cellulose, microcrystalline cellulose and mixtures thereof.

10. The procedure according to claim 6, **characterized in that** said procedure is carried out at room temperature.

11. The procedure according to claim 6, **characterized in that** the coating hydrophobic composition comprises compounds selected from the group comprised by silicon dioxide, calcium orthophosphate, calcium carbonate, magnesium carbonate, aluminum oxide, coloidal silica.

12. The procedure according to claim 6, **characterized in that** after stage ii) a grinding stage may be incorporated for the obtained mixture.

13. A procedure for the obtention of tablets for topic use, **characterized in that** it comprises compressing a nixture which comprises from 1 to 16 w% of the particles according to claim 1 and from 99 to 84 wt% of excipients, wherein said tablets friability is equal to 1.2% or less and their velocity of disintegration in water is equal to 30 seconds or less.

14. The procedure according to claim 13, **characterized in that** excipients are selected from the group comprised of carboxy methyl cellulose, lactose, microcrystalline cellulose, sodium croscarmellose and mixtures thereof.

15. The procedure according to claim 13, **characterized in that** the tablet is a personal hygiene tablet.

16. The procedure according to claim 13, **characterized in that** the tablet is a cosmetic use tablet.

17. The procedure according to claim 13, **characterized in that** the tablet is a pharmacological tablet.

18. Solid compressible particles **characterized in that** they comprise from 50 to 70 wt% of a crystalline alcohol, from 25 to 45 wt% of a cellulose polymer and from 5 to 18 w% of a coating hydrophobic compound.

19. The particle according to claim 18, **characterized in that** the crystalline alcohol is selected from the group comprised of polyols, terpenes and menthol.

20. The particle according to claim 19, **characterized in that** the polyol is selected from the group comprised of xylitol, isomaltose, sorbitol, maltitol, starch hydrolisates.

21. The particle according to claim 19, **characterized in that** the terpene is selected from the group comprised of thymol, carvacrol and menthol.

22. The particle according to claim 18, **characterized in that** the cellulose polymer is selected from the group comprised of carboxy methyl cellulose, microcrystalline cellulose, sodium croscarmellose and mixtures thereof.

23. The particle according to claim 18, **characterized in that** the coating hydrophobic composition comprises compounds selected from the group comprised of silicon dioxide, calcium carbonate, aluminum oxide and coloidal silica.

24. A procedure for the obtention of the compressible solid particles according to claim 18, **characterized in that** it comprises the stages of:
iv) contacting said crystalline alcohol with a coating hydrophobic composition;
v) mixing of both components;
vi) addition of a cellulose polymer thereto and further mixing; and
vii) sieving of the compressible solid particles, wherein said particles size is of 180 microns or less.

25. The procedure according to claim 24, **characterized in that** the crystalline alcohol is selected from the group comprised of polyols, terpenes and menthol.

26. The procedure according to claim 25, **characterized in that** the polyol is selected from the group comprised of xylitol, isomaltose, sorbitol, maltitol and starch hidrolisates.

27. The procedure according to claim 25, **characterized in that** the terpene is selected from the group comprised of thymol, carvacrol and menthol.

28. The procedure according to claim 24, **characterized in that** the cellulose polymer is selected from the group comprised of carboxy methyl cellulose, microcrystalline cellulose, sodium croscarmellose and mixtures thereof.

29. The procedure according to claim 24, **characterized in that** said procedure is performed at room temperature.

30. The procedure according to claim 24, **characterized in that** the coating hydrophobic composition comprises compounds selected from the group comprising silicon dioxide, calcium orthophosphate, magnesium carbonate, and aluminum oxide.

31. The procedure according to claim 24, **characterized in that** previous to stage I) a grinding and sieving of the crystalline alcohol may be carried out.

32. A procedure for the obtention of topic use tablets, **characterized in that** it comprises compression of a mixture comprising from 1 to 16 wt% of particles according to claim 18 and from 99 to 84 wt% of excipients, wherein said tablets exhibit a friability of 1.2 or less and a disintegration speed in water of 30 seconds or less.

33. The procedure according to claim 32, **characterized in that** excipients are selected from the group comprised of carboxy methyl cellulose, lactose, microcrystalline cellulose, sodium croscarmellose and mixtures thereof.

34. The procedure according to claim 32, **characterized in that** the tablet is a tablet for personal hygiene.

35. The procedure according to claim 32, **characterized in that** the tablet is a tablet for cosmetic use.

36. The procedure according to claim 32, **characterized in that** the tablet is a tablet for pharmacological use.

37. Use of compressible particles according to claims 1 and 18, **characterized in that** said particles are used for the elaboration of tablets.
